# EUROPÄISCHE PATENTSCHRIFT

(11) **EP 0 962 185 B1**
(45) Veröffentlichungstag und Bekanntmachung des Hinweises auf die Patenterteilung: **11.08.2004**
(21) Anmeldenummer: 99109701.5
(22) Anmeldetag: 17.05.1999
(51) Int. Cl.: A61B 5/00, G01N 21/64

(54) **Vorrichtung zum Erkennen von Karies, Plaque, Konkrementen oder bakteriellem Befall an Zähnen**
Apparatus for determining caries, plaque, concrements or bacterial decay of teeth
Dispositif destiné à la détermination de la cariée, de la plaque, de concrétions ou d' affectations bactérielles des dents

(30) Priorität: 04.06.1998 DE 19825021
(43) Veröffentlichungstag der Anmeldung: 08.12.1999
(73) Patentinhaber: Kaltenbach & Voigt GmbH & Co. KG, 88400 Biberach/Riss (DE)
(72) Erfinder: Hibst, Raimund, Dr., 89155 Erbach (DE); Paulus, Robert, 86368 Gersthofen (DE)
(74) Vertreter: Schmidt-Evers, Jürgen, Dipl.-Ing.

(56) Entgegenhaltungen:
- WO-A-84/04665
- DE-A- 3 345 465
- DE-A- 19 541 686
- DE-U- 9 317 984
- DE-U- 9 404 312
- DE-U- 29 705 934
- US-A- 4 479 499
- US-A- 4 836 206

## Beschreibung

Die vorliegende Erfindung betrifft eine Vorrichtung zum Erkennen von Karies, Plaque, Konkrementen, bakteriellem Befall usw. an Zähnen bzw. ein Verfahren zum Messen der Fluoreszenzaktivität von Zähnen.

Es ist bekannt, Karies, Plaque oder bakteriellen Befall an Zähnen durch visuelle Untersuchung oder durch Verwendung von Röntgenstrahlung zu entdecken. Mit Hilfe einer visuellen Untersuchung lassen sich jedoch häufig keine zufriedenstellenden Ergebnisse erzielen, da sich beispielsweise Karies im Frühstadium oder an einem schwer einsehbaren Zahnbereich nicht feststellen lässt. Obwohl sich andererseits Röntgenstrahlen als sehr wirksame Art zur Feststellung eines Kariesbefalls oder anderer Zahnkrankheiten herausgestellt haben, ist auch dieses Untersuchungsverfahren aufgrund der schädigenden Wirkung der Röntgenstrahlen für die menschliche Gesundheit nicht optimal. Es bestand daher das Bedürfnis nach der Entwicklung einer neuen Technik, um das Vorhandensein von Karies an Zähnen feststellen zu können.

In der DE 30 31 249 C2 wurde ein berührungsloses Untersuchungsverfahren zum Feststellen von Karies an menschlichen Zähnen vorgeschlagen, wobei der Zahn mit nahezu monochromatischem Licht bestrahlt wird. Die annähernd monochromatische Lichtstrahlung regt an dem Zahn eine Fluoreszenzstrahlung an. Dabei wurde entdeckt, dass das von dem Zahn reflektierte Fluoreszenzspektrum deutliche Unterschiede zwischen kariösen und gesunden Zahnbereichen aufweist. So ist im roten Spektralbereich des Fluoreszenzspektrums des Zahnes (d. h. zwischen 550 nm und 650 nm) die Intensität deutlich höher als bei einem gesunden Zahn. Dagegen ist im blauen Spektralbereich des reflektierten Fluoreszenzspektrums des Zahnes (d. h. zwischen 350 nm und 450 nm) die Intensität der Fluoreszenzstrahlung für kariöse Bereiche und gesunde Bereiche des Zahnes nahezu identisch. In der DE 30 31 249 C2 wurde daher vorgeschlagen, den Zahn mit einer Wellenlänge von 410 nm zu bestrahlen und mittels zweier Filter die Fluoreszenzstrahlung des Zahnes für eine erste Wellenlänge von 450 nm sowie eine zweite Wellenlänge von 610 nm, d. h. im blauen und roten Spektralbereich, beispielsweise mit Hilfe von Photodetektoren zu erfassen. Die durch diese Anordnung erfassten Fluoreszenzstrahlungintensitäten werden subtrahiert, so dass aufgrund der dadurch gewonnenen Differenzintensität ein gesunder Zahnbereich eindeutig von einem kariösen Zahnbereich unterschieden werden kann.

Die DE 4200741 A1 schlägt als vorteilhafte Weiterbildung vor, die Fluoreszenz des Zahnes durch eine Anregungsstrahlung mit einer Wellenlänge im Bereich 360 nm bis 580 nm hervorzurufen und die am bestrahlten Zahn hervorgerufene Fluoreszenzstrahlung im Wellenlängenbereich zwischen 620 nm und 720 nm auszufiltern. Durch diese Maßnahme wird erzielt, dass der Abstand zwischen der Wellenlänge der Anregungsstrahlung und der empfangenen Fluoreszenzstrahlung ausreichend groß ist, so dass die Anregungsstrahlung nicht die Auswertungsergebnisse durch Überlagerung der Fluoreszenzstrahlung verfälschen kann.

Den zuvor beschriebenen bekannten Untersuchungsverfahren bzw. Vorrichtungen ist gemeinsam, dass zur Anregung der Fluoreszenz an einem zu untersuchenden Zahn eine Anregungsstrahlung mit einer relativ kurzen Wellenlänge, d. h. kleiner als 580 nm, verwendet wird. Dadurch kann zwar einerseits ein verhältnismäßig hoher Wirkungsquerschnitt für die Erzeugung der Fluoreszenzstrahlung erzielt werden, jedoch ist die Fluoreszenzstrahlung für gesundes Zahngewebe erheblich stärker als die für kariöse Läsionen. Daher ist bei den zuvor beschriebenen Untersuchungsverfahren ein aufwendiger direkter Vergleich der in einem bestimmten Wellenlängenbereich von benachbarten gesunden und kariösen Bereichen emittierten Fluoreszenzstrahlung notwendig oder es müssen die Messsignale der in zwei unterschiedlichen Wellenlängenbereichen erfassten Fluoreszenzstrahlung aufwendig miteinander verglichen werden.

In der DE 195 41 686 A1, die wie die DE 42 00 741 A1 von der Anmelderin der vorliegenden Anmeldung stammt, wurde daher vorgeschlagen, zur Anregung der Fluoreszenz an einem zu untersuchenden Zahn eine Anregungsstrahlung mit einer Wellenlänge zwischen 600 nm und 670 nm zu verwenden. Zur Erfassung der an dem bestrahlten Zahn angeregten Fluoreszenzstrahlung wird eine Spektralfilteranordnung eingesetzt, welche Fluoreszenzstrahlung mit einer Wellenlänge zwischen 670 nm und 800 nm durchläßt, d.h. gemäss der DE 195 41 686 A1 wird nur Fluoreszenzstrahlung mit einer Wellenlänge zwischen 670 nm und 800 nm für die Erkennung von Karies, Plaque oder bakteriellen Befall an dem bestrahlten Zahn ausgewertet.

Mit Hilfe der in der DE 195 41 686 A1 vorgeschlagenen Maßnahmen wird eine verbesserte Empfindlichkeit der Kariesdetektion erzielt. Die Anregung der Fluoreszenzstrahlung mit einer Anregungsstrahlung in dem zuvor beschriebenen Wellenlängenbereich zwischen 600 nm und 670 nm weist den Vorteil auf, dass die Fluoreszenzstrahlung von gesunden Zahnbereichen bei derartigen Anregungswellenlängen stark abnimmt, so dass die Fluoreszenzstrahlung von kariösen Bereichen nur noch gering von der Autofluoreszenz des gesunden Zahngewebes überlagert wird und Karies, Plaque oder bakterieller Befall an Zähnen einfach, störunanfällig und mit hoher Empfindlichkeit erkannt werden kann.

Bei einer in der DE 297 05 934 U 1 beschriebenen optischen Diagnosevorrichtung, die mit einem zahnärztlichen Behandlungslaser kombiniert ist, werden vergleichbare Wellenlängen für die Anregungsstrahlung sowie für die untersuchte Fluoreszenzstrahlung verwendet. Genau genommen wird ein zu untersuchender und ggf. zu behandelnder Zahn mit einer Strahlung im Bereich von 600 bis 700 nm bestrahlt und die Fluoreszenzstrahlung im Bereich zwischen 650 und 850 nm ausgewertet.

Schließlich beschreibt auch WO 84/04665 A1 eine optische Diagnosevorrichtung, die allerdings zur Erkennung von karzinogenem Gewebe vorgesehen ist. Hierbei wird das zu untersuchende Gewebe einer Anregungsstrahlung von ca. 620 nm bis ca. 640 nm ausgesetzt und die Fluoreszenzstrahlung im Bereich zwischen 900 nm und 1300 nm ausgewertet.

Ausgehend von dem zuvor beschriebenen bekannten Stand der Technik liegt der vorliegenden Erfindung die Aufgabe zugrunde, die Detektionsempfindlichkeit bei Untersuchen der Fluoreszenzeigenschaften eines Zahnes bzw. bei der Erkennung von Karies, Plaque, Konkrementen oder bakteriellem Befall an Zähnen weiter zu erhöhen, d. h. den Unterschied zwischen Messsignalen von gesunden Zahnbereichen einerseits und kariösen Zahnbereichen andererseits zu erhöhen.

Diese Aufgabe wird gemäss der vorliegenden Erfindung durch ein Verfahren mit den Merkmalen des Anspruches 1 bzw. eine Vorrichtung mit den Merkmalen des Anspruches 5 gelöst. Die Unteransprüche beschreiben bevorzugte und vorteilhafte Ausführungsformen der vorliegenden Erfindung, die ihrerseits zu einer verbesserten Empfindlichkeit oder zu einem möglichst einfachen Aufbau der erfindungsgemäßen Vorrichtung beitragen.

Gemäss der vorliegenden Erfindung wird vorgeschlagen, den zu untersuchenden Zahn mit einer Wellenlänge zischen 680 nm und 800 nm anzuregen und die Fluoreszenzstrahlung mit einer Wellenlänge oberhalb von ca. 850 nm auszuwerten. Nach den der vorliegenden Patentanmeldung zugrundeliegenden Erkenntnissen lässt sich mit Hilfe dieser Maßnahme eine besonders empfindliche Erfassung von versteckter Karies (beispielsweise in Fissuren oder approximalen Zahnbereichen) erreichen, da in diesem Wellenlängenbereich der Fluoreszenzstrahlung der Anteil von kariesspezifischen Fluorophoren und anderen Ablagerungen besonders hoch ist, aber gesunder Zahnschmelz oder Dentin nicht oder nur sehr gering fluoreszieren.

Bei der Entwicklung der vorliegenden Erfindung wurde entdeckt, dass offenbar ober- und unterhalb einer bestimmten Anregungswellenlänge verschiedene Gruppen von Fluorophoren erfaßt werden, so dass für eine Differenzierung (z. B. von Dekalzifizierungen und Läsionen mit organischen Einlagerungen oder bakteriellem Befall) auch die Kombination verschiedener Nachweisbereiche, d. h. verschiedener ausgewerteter Wellenlängenbereiche der Fluoreszenzstrahlung, und/oder Anregungswellenlängen vorteilhaft ist.

Die vorliegende Erfindung wird nachfolgend anhand bevorzugter Ausführungsbeispiele unter Bezugnahme auf die beigefügte Zeichnung näher erläutert.
Fig. 1 zeigt ein erstes Ausführungsbeispiel der erfindungsgemäßen Vorrichtung zum Erkennen von Karies, Plaque, Konkrementen oder bakteriellem Befall an Zähnen,
Fig. 2 zeigt ein zweites Ausführungsbeispiel der erfindungsgemäßen Vorrichtung,
Fig. 3 zeigt eine beispielhafte Querschnittsansicht eines Lichtleiters der in Fig. 1 oder Fig. 2 gezeigten erfindungsgemäßen Vorrichtung,
Fig. 4a-4c zeigen verschiedene Ausgestaltungen einer Lichtleitersonde der in Fig. 1 oder Fig.2 gezeigten erfindungsgemäßen Vorrichtung,
Fig. 5 zeigt eine Darstellung der Fluoreszenzstrahlung abhängig von der Anregungswellenlänge bei gesundem Zahnschmelz, wobei die Fluoreszenzstrahlung über unterschiedliche Wellenlängenbereiche integriert worden ist,
Fig. 6 zeigt eine Darstellung der Fluoreszenzstrahlung abhängig von der Anregungswellenlänge für einen kariösen Zahnbereich, wobei die Fluoreszenzstrahlung für verschiedene Wellenlängenbereiche integriert worden ist,
Fig. 7 zeigt eine zusammenfassende Darstellung der in Fig. 5 und Fig. 6 gezeigten Meßergebnisse,
Fig. 8 zeigt eine Darstellung der Intensität der Fluoreszenzstrahlung abhängig von der Fluoreszenzwellenlänge bei einer Anregungswellenlänge von 633 nm für einen kariösen Zahnbereich und Zahnschmelz,
Fig. 9 zeigt eine Darstellung der Intensität der Fluoreszenzstrahlung abhängig von der Fluoreszenzwellenlänge bei einer Anregungswellenlänge von 780 nm für einen kariösen Zahnbereich und Zahnschmelz,
Figur 10a und 10b zeigen Darstellungen des Verhältnisses der Fluoreszenzstrahlung eines kariösen Zahnbereichs zu der Fluoreszenzstrahlung eines gesunden Zahnbereichs für unterschiedliche Anregungswellenlängen und unterschiedliche ausgewertete Wellenlängen der Fluoreszenzstrahlung, und
Fig. 11 zeigt eine Gegenüberstellung der Transmissionsgrade von zwei Filtern, die bei Untersuchungen eingesetzt wurden.

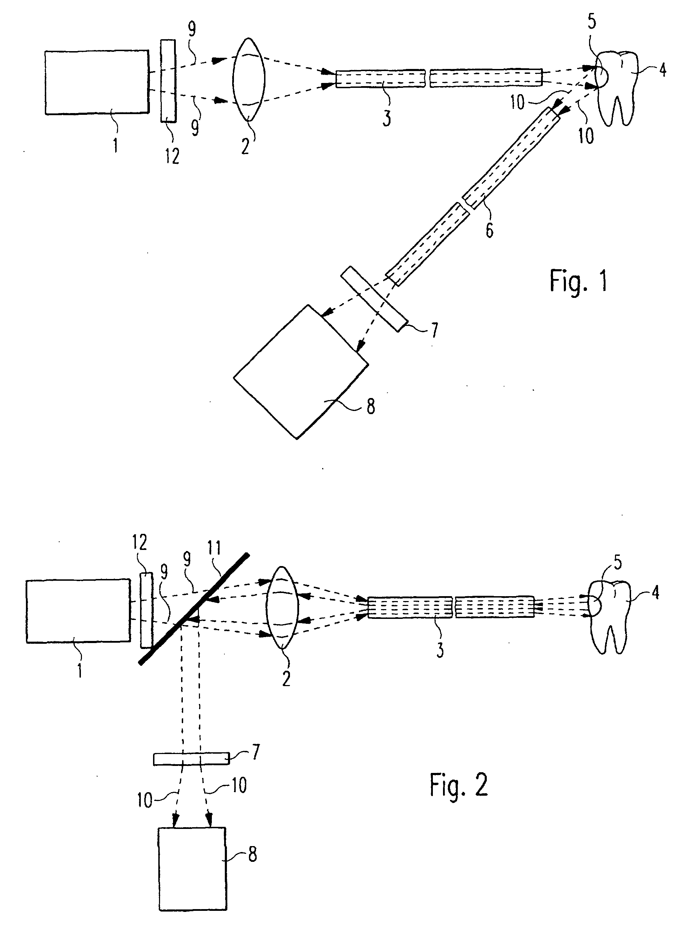

Figur 1 zeigt ein erstes Ausführungsbeispiel einer erfindungsgemäßen Vorrichtung zum Erkennen von Karies, Plaque, Konkrementen oder bakteriellem Befall an Zähnen. Eine Lichtquelle 1 erzeugt eine Anregungsstrahlung 9, die über ein Einkopplungslinsensystem 2 und einen Lichtleiter einem zu untersuchenden Bereich 5 eines Zahnes 4 zugeführt wird. Die Anregungsstrahlung liegt beispielsweise zwischen 680 nm und 800 nm und beträgt vorzugsweise ca. 780 nm. Durch die Anregungsstrahlung wird an dem bestrahlten Zahnbereich 5 eine Fluoreszenzstrahlung 10 über einen relativ breiten Spektralbereich hervorgerufen, die über einen zweiten Lichtleiter 6 und ein Spektralfilter 7 einer Erfassungseinrichtung 8 zum Erfassen und Auswerten der Fluoreszenzstrahlung des Zahnes zugeführt wird. Das Spektralfilter ist derart ausgestaltet, daß es nur für eine Fluoreszenzstrahlung mit einer Wellenlänge größer als ca. 850 nm durchlässig ist. Die Erfassungseinrichtung 8 wertet die ihr zugeführte Fluoreszenzstrahlung 10 direkt aus und schließt unmittelbar aus der erfaßten Fluoreszenzstrahlung 10 auf das Vorhandensein bzw. Nichtvorhandensein von Karies, Plaque oder bakteriellem Befall an dem bestrahlten Zahnbereich 5.

Die Lichtquelle 1 ist vorzugsweise ein HeNe-Laser oder eine Laserdiode, die die Anregungsstrahlung in dem oben beschriebenen bevorzugten Wellenlängenbereich erzeugt.

Untersuchungen haben gezeigt, daß die als Lichtquelle 1 dienenden und getesteten Laserdioden zusätzlich zur jeweiligen Laserlinie auch einen Untergrund langwelliger Strahlung emittieren, wobei diese langwellige Strahlung zusammen mit der eigentlich zu detektierenden Fluoreszenzstrahlung das der Erfassungseinrichtung 8 vorgeschaltete Spektralfilter 7 passieren und somit die Messung verfälschen kann. Diese langwellige Untergrundstrahlung kann mit Hilfe eines der Lichtquelle bzw. Laserdiode 1 nachgeschalteten Kurzpaßfilters 12 eliminiert werden, dessen seinen Durchlaßbereich definierende Kantenwellenlänge vorteilhafterweise zwischen der eigentlichen Anregungswellenlänge der Laserdiode bzw. Lichtquelle 1 und dem kurzwelligen Ende des jeweils gewählten Nachweisfensters derart liegt, daß die Anregungsstrahlung 9 das Kurzpaßfilter 12 ungehindert passieren kann, jedoch die langwellige

Untergrundstrahlung zuverlässig über den gesamten Spektralbereich der nachgewiesenen Fluoreszenzstrahlung ausgefiltert wird.

Die das Kurzpaßfilter 12 passierende Anregungsstrahlung 9 wird über ein separates Linsensystem 2 oder eine bei Laserdioden häufig schon integrierte Kollimatoroptik in den Lichtleiter 3 eingekoppelt. Ein solcher Lichtleiter kann starr oder flexibel ausgeführt und zudem an seinem dem Zahn zugewandten Ende mit weiteren optischen Mitteln (Linsen) zur gezielten Strahlführung ausgestattet und/oder in seinen Abmessungen an dem Mundbereich des Patienten sowie an den zu untersuchenden Zahn angepaßt sein. Des weiteren können an oder in dem Lichtleiter 3 austauschbare Ablenkspiegel oder Linsen angebracht sein, die eine Untersuchung des Zahnes 4 erleichtern. Die Verwendung des Lichtleiters 3 ermöglicht es also, die Anregungsstrahlung 9 gezielt dem zu untersuchenden Bereich 5 des Zahnes bzw. der Zähne 4 zuzuführen. Dadurch kann die erfindungsgemäße Vorrichtung flexibel an verschiedene Erfordernisse in der täglichen Praxis bei der Karieserkennung an Zähnen eines Patienten angepaßt werden. Das zu dem Lichtleiter 3 Gesagte gilt im gleichen Maße auch für den weiteren Lichtleiter 6, der die Fluoreszenzstrahlung dem Spektralfilter 7 zuführt. Die beiden Lichtleiter 3 und 6 können jeweils mehrere Lichtleiterfasern aufweisen. Bei Verwendung eines Lasers als Lichtquelle 1 kann die Anregungsstrahlung 9 sowie die Fluoreszenzstrahlung 10 über relativ dünne Lichtleiterfasern mit einem Kerndurchmeser von beispielsweise 200 µm übertragen werden. Die Verwendung zweier getrennter Lichtleiter 3 und 6 für die Übertragung der Anregungsstrahlung 9 bzw. der Fluoreszenzstrahlung 10 ist insbesondere für die Untersuchung von Außenflächen eines Zahnes vorteilhaft. Bei dem in Figur 1 dargestellten Ausführungsbeispiel kann somit die Position der Lichtleiter 3 und 6 unabhängig voneinander und individuell am Zahn gewählt werden, was im Einzelfall eine Optimierung der Nachweisempfindlichkeit für tiefliegende oder sehr versteckt liegende Läsionen ermöglicht.

Das Spektralfilter 7 der erfindungsgemäßen Vorrichtung kann beispielsweise durch ein Farbglas-Kantenfilter oder andere optische Elemente zur spektralen Selektion, z. B. ein Beugungsgitter, Spektrometer usw. realisiert sein. Das Filter ist vorteilhafterweise derart ausgestaltet, daß es selber möglichst wenig fluoresziert.

Die Erfassungseinrichtung 8 weist vorteilhafterweise als lichtempfindliche Elemente Photodioden zur Detektion der Fluoreszenzstrahlung auf. Zur Erhöhung der Empfindlichkeit können die Photodioden mit einem integrierten Vorverstärker ausgestattet sein. Ebenso kommt als Verstärkerelement in dem optischen Feld der Fluoreszenzstrahlung 10 ein Photomultiplierer in Frage.

Für den Fall, daß sowohl die Lichtquelle 1 als auch das lichtempfindliche Element der Erfassungseinrichtung 8 aus einem Halbleiterbauelement bestehen, kann zur Spannungsversorgung der erfindungsgemäßen Vorrichtung ein Niederspannungsnetzteil eingesetzt werden, welches aufgrund der geringen Leistungsaufnahme lediglich aus Batterien oder Akkumulatoren bestehen könnte.

Figur 2 zeigt ein zweites Ausführungsbeispiel einer erfindungsgemäßen Vorrichtung zum Erkennen von Karies, Plaque oder bakteriellem Befall, wobei die in Figur 1 bereits dargestellten Bestandteile der Vorrichtung durch identische Bezugszeichen versehen sind. Bei dem in Figur 2 dargestellten Ausführungsbeispiel wird die Fluoreszenzstrahlung 10 über denselben Lichtleiter wie die Anregungsstrahlung 9 übertragen. Zum Auskoppeln der Fluoreszenzstrahlung 10 aus dem Strahlengang in dem Lichtleiter 3 ist ein Strahlteiler 11 vorgesehen, der zwischen der Lichtquelle 1 und der Linsenanordnung 2 oder alternativ zwischen der Linsenanordnung 2 und dem lichtquellenseitigen Ende des Lichtleiters 3 angeordnet ist. Diese Ausgestaltung der vorliegenden Erfindung ist insbesondere für den Einsatz zur Untersuchung von Wurzelkanälen von Bedeutung.

Die Anregungsstrahlung 9 sowie die Fluoreszenzstrahlung 10 können - wie bereits anhand des in Figur 1 dargestellten Ausführungsbeispiels erläutert worden ist - jeweils über mehrere Lichtleiterfasern des Lichtleiters 3 übertragen werden. Um die Detektionssicherheit und Detektionsgenauigkeit zu erhöhen bzw. zu stabilisieren kann in diesem Fall der Lichtleiter 3 eine oder mehrere zentral angeordnete Lichtleiterfasern zur Übertragung der Anregungsstrahlung 9 und mehrere konzentrisch um diese zentrale(n) Lichtleiterfaser(n) angeordnete Lichtleiterfasern für die Übertragung der Fluoreszenzstrahlung 10 umfassen. An dem der Erfassungseinrichtung 8 zugewandten Ende des Lichtleiters 3 können die Detektionsfasern, welche die am Zahn 4 angeregte Fluoreszenzstrahlung übertragen, zur Verbesserung der Detektionsgenauigkeit gebündelt sein. Durch Anschrägen der äußeren Lichtleiterfasern, die zur Übertragung der Fluoreszenzstrahlung 10 vorgesehen sind, kann zudem eine sichere und weitreichende Überlappung der Anregungsstrahlung 9 und der Fluoreszenzstrahlung 10 erzielt werden, was zu einer weiteren Verbesserung der Detektionsgenauigkeit beiträgt.

Bei den beiden in Figur 1 und 2 dargestellten Ausführungsbeispielen kann die Erfassungseinrichtung 8 mit einer (nicht gezeigten) Anzeigeeinrichtung gekoppelt sein, die das von der Erfassungseinrichtung 8 gelieferte Meßsignal visuell darstellt. Ebenso ist eine akustische Anzeige des Meßsignals denkbar.

Untersuchungen haben ergeben, daß insbesondere Lichtleiter aus Kunststoff oder Glas im gemäß der vorliegenden Erfindung genutzten Spektralbereich fluoreszieren können (bei den relativ teueren Quarzlichtleitern ist hingegen die Fluoreszenzstrahlung vergleichsweise gering). Dies hat zur Folge, daß sowohl in der Anregungsfaser als auch in den Detektionsfasern Fluoreszenzstrahlung hervorgerufen werden kann, die zusammen mit der am Zahn hervorgerufenen Fluoreszenzstrahlung das Spektralfilter 7 passieren und somit das Meßergebnis verfälschen kann. Insbesondere bei spiegelnden Oberflächen kann das von den Fasern selbst erzeugte Fluoreszenzsignal zu einem erheblichen Störfaktor werden, den es zu vermeiden gilt.

Um dieses Problem zu beheben, können - wie bereits zuvor erwähnt worden ist - nicht fluoreszierende Lichtleiter, vorzugsweise aus Quarz, verwendet werden. Da diese Lichtleiter jedoch sehr teuer sind, kommt diese Alternative wohl nur für Einfasersysteme in Betracht.

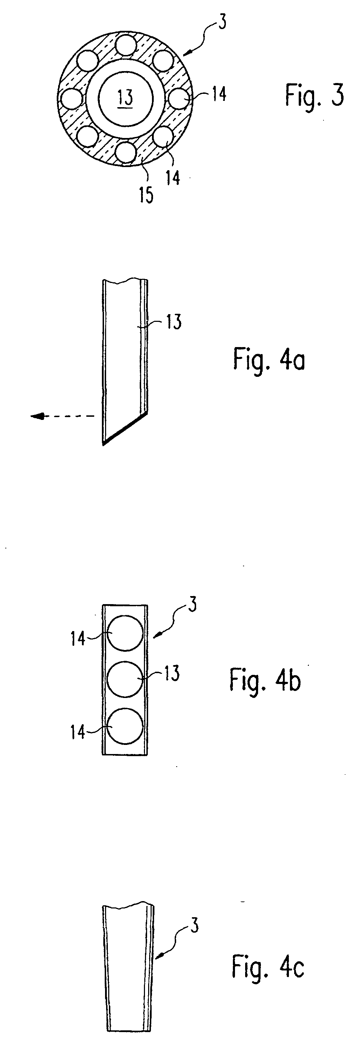

Eine weitere Möglichkeit ist der Einsatz von Filtern, die diese von den Lichtleitern erzeugte Fluoreszenzstrahlung ausfiltern. Wie bereits anhand Fig. 2 erläutert worden ist, können so z.B. die Anregungsfasern mit einem Kurzpaßfilter 12 kombiniert werden, das lediglich die Anregungsstrahlung 12 durchläßt, jedoch langwellige Fluoreszenzstrahlung blockiert. Die Detektionsfasern, welche die am Zahn 4 angeregte Fluoreszenzstrahlung 10 der Erfassungseinrichtung 8 zuführen, können hingegen mit mindestens einem Langpaßfilter gekoppelt werden, das somit in dem Strahlungspfad zwischen dem Zahn 4 und der Erfassungseinrichtung 8 anzuordnen ist. Dieses Langpaßfilter ist derart auszugestalten, daß es die am Zahn 4 angeregte Fluoreszenzstrahlung 10 durchläßt, nicht jedoch das gestreute Anregungslicht, welches wegen seiner vergleichsweise hohen Intensität in den Detektionsfasern einen störenden Pegel an Faser-Fluoreszenzstrahlung erzeugt. Dieses Langpaßfilter kann zusätzlich zu dem bereits beschriebenen Spektralfilter 7 oder anstatt des Spektralfilter 7 vor der Erfassungseinrichtung 8 eingesetzt werden und besitzt einen Durchlaßbereich für Wellenlängen oberhalb der eigentlichen Anregungswellenlänge, insbesondere für Wellenlängen oberhalb von ca. 800 nm. Fig. 3 zeigt einen Lichtleiter 3 mit einer besonders vorteilhafte Kombination einer nicht fluoreszierenden Anregungsfaser 13 zur Übertragung der Anregungsstrahlung 9 mit mehreren konzentrisch angeordneten Detektionsfasern 14 zur Erfassung und Übertragung der Fluoreszenzstrahlung 10, wobei die Detektionsfasern 14 mit dem zuvor beschriebenen Filter bzw. den Filtern versehen sind, die bei dem in Fig. 3 gezeigten Ausführungsbeispiel durch einen Filterglasring 15 realisiert sind.

Weitere Untersuchungen haben gezeigt, daß auch Konkremente an Zahnwurzeln zu sehr deutlichen Fluoreszenzsignalen führen und demnach mit Hilfe der vorliegenden Erfindung einfach zu detektieren sind. Um das erfindungsgemäße Verfahren bei der Therapiekontrolle auch in der Paradontologie vorteilhaft einsetzen zu können, ist es wichtig, daß die Sonde bzw. der Applikator mit dem Lichtleiter in die Paradontaltasche eingeführt werden kann. Dies kann beispielsweise dadurch gewährleistet sein, daß als Lichtleiter 3 für die Übertragung der Anregungsstrahlung 9 lediglich eine in Fig. 4a gezeigte Einzelfaser verwendet wird, deren Ende abgeschrägt und verspiegelt ist, so daß das Anregungslicht aus dieser Faser seitlich austreten und die Wurzeloberfläche gezielt beleuchten kann. Alternativ können, wie in Fig. 4b gezeigt ist, die Anregungs- und Detektionsfasern 13 bzw. 14 linear am zahn- bzw. applikationsseitigen Ende des Lichtleiters 3 angeordnet sein, um auf diese Weise eine möglichst exakte Bestrahlung der Zahnwurzeln und Detektion der dort angeregten Fluoreszenz zu ermöglichen. Fig. 4c zeigt schließlich die Verwendung eines Lichtkeils für die Übertragung der Anregungsstrahlung 9, der ebenfalss einfach in die Paradontaltasche eingeführt werden kann.

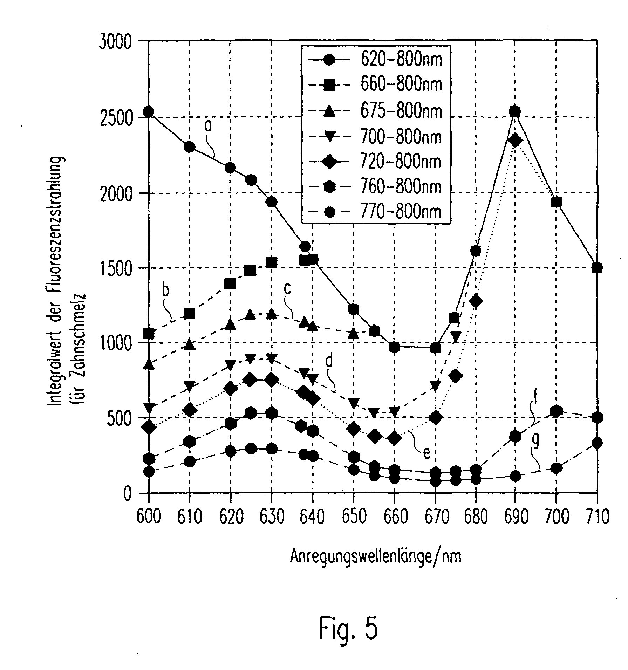

Die der vorliegenden Erfindung zugrundeliegende Erkenntnis, d. h. die Detektionsempfindlichkeit und Detektionsgenauigkeit dadurch zu erhöhen, daß die am Zahn angeregte Fluoreszenzstrahlung lediglich für Wellenlängen größer als ca. 800 nm, insbesondere für Wellenlängen größer als ca. 820 nm, ausgewertet wird, beruht auf experimentiellen Ergebnissen, die nachfolgend näher anhand Figur 5-10 erläutert werden sollen.

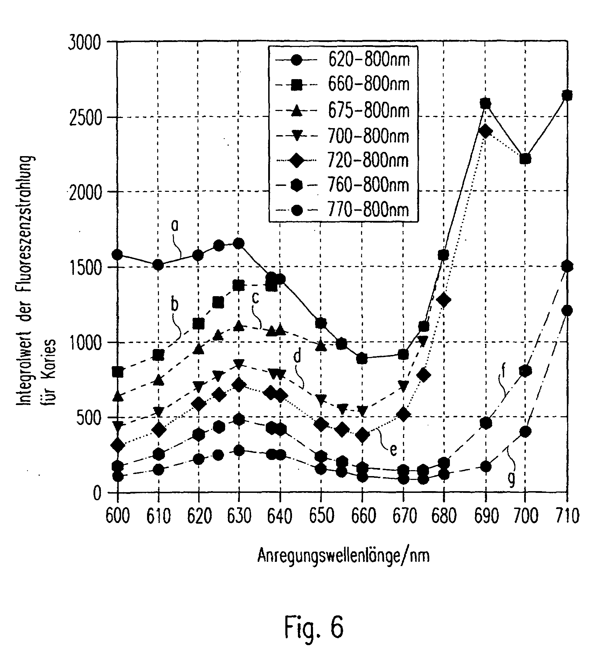

Um die Detektionsempfindlichkeit weiter zu erhöhen, d. h. den Unterschied zwischen den Meßsignalen von gesunden und kariösen Zahnbereichen zu steigern, wurden für jeweils drei Proben von gesundem Zahnschmelz, Dentin und Karies die angeregten Fluoreszenz-Emissionsspektren gemessen, wobei diese Messung für insgesamt 16 verschiedene Anregungswellenlängen im Bereich von 600 nm bis 710 nm durchgeführt wurden. Die Fluoreszenz-Emissionsspektren als solche sowie ihre Variation mit der Anregungswellenlänge sind insgesamt bei gesundem und kariösem Gewebe sehr ähnlich. Um zu bestimmen, welche Unterscheidungsmöglichkeiten die Detektion mit verschiedenen Grenzwellenlängen für die Auswertung der Fluoreszenzstrahlung, d. h. mit verschiedenen Kantenfiltern, bietet, wurde die Intensität in den Fluoreszenzspektren ausgehend von unterschiedlichen Wellenlängen der Fluoreszenzstrahlung bis zu der Fluoreszenzstrahlungswellenlänge 800 nm aufintegriert. Die Ergebnisse dieser Messungen sind in Figur 5 und 6 dargestellt, wobei Figur 5 den Integralwert des jeweiligen Fluoreszenzstrahlungswellenlängenbereichs für Zahnschmelz und Figur 6 den Integralwert des jeweiligen Fluoreszenzstrahlungswellenlängenbereichs für kariöse Zahnbereiche darstellt. Die Kurve a) entspricht dem Integralwert für den Wellenlängenbereich der Fluoreszenzstrahlung von 620 nm bis 800 nm, während die Kurve b) jeweils dem Fluoreszenzstrahlungswellenlängenbereich 660 nm - 800 nm entspricht. Die Kurve c) ist dem Fluoreszenzstrahlungswellenlängenbereich 675 nm bis 800 nm zugeordnet, während die Kurve d) den Fluoreszenzstrahlungswellenlängenbereich 700 nm bis 800 nm darstellt. Der Kurvenverlauf e) entspricht dem Wellenlängenbereich 720 nm bis 800 nm, die Kurve f) entspricht dem Wellenlängenbereich 760 nm - 800 nm und die Kurve g) entspricht dem Wellenlängenbereich 770 nm - 800 nm der Fluoreszenzstrahlung.

Der Vergleich der in Figur 5 und 6 dargestellten Diagramme zeigt in dem aus der DE 19541686 A1 bekannten Bereich der Anregungswellenlängen zwischen 600 nm und 670 nm für keinen der getesteten Integralbereiche besonders große Unterschiede zwischen der am Zahnschmelz angeregten Fluoreszenzstrahlung und der an kariösen Bereichen angeregten Fluoreszenzstrahlung. Derartige deutliche Unterschiede treten erst bei relativ großen Anregungswellenlängen oberhalb von 680 nm, insbesondere oberhalb von 700 nm, auf, wobei deutliche Unterschiede insbesondere dann zu erkennen sind, wenn der Nachweisbereich auf langwellige Fluoreszenzstrahlung beschränkt ist (vgl. die Kurvenverläufe f) und g)).

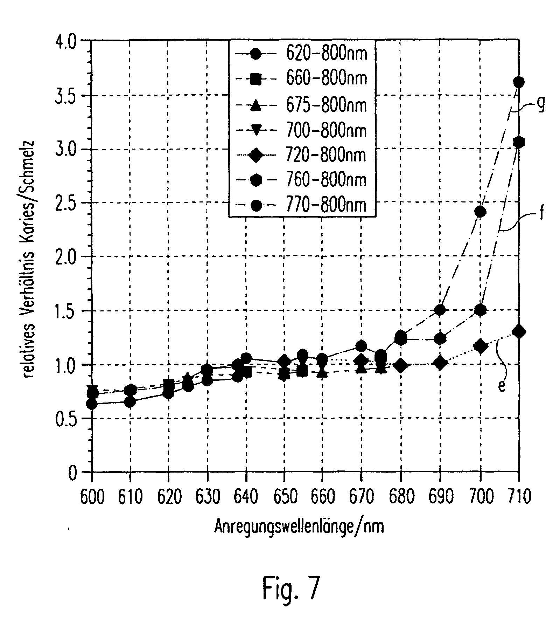

Die anhand Fig. 5 und 6 erläuterten Meßergebnisse sind in Fig. 7 zusammengefaßt, wobei für die bereits in Fig. 5 und 6 gezeigten Integralbereiche der Fluoreszenzwellenlänge jeweils das relative Verhältnis der für kariöse Zahnbereiche detektierten Fluoreszenzstrahlung und der an Zahnschmelz angeregten Fluoreszenzstrahlung abhängig von der Anregungswellenlänge dargestellt ist. Insbesondere sind in Fig. 7 wiederum die Kurvenverläufe f) und g) dargestellt, die den in Fig. 5 und 6 gezeigten Kurven f) und g) entsprechen und einer Integralerfassung der am Zahn angeregten Fluoreszenzstrahlung für den Fluoreszenzwellenlängenbereich 760 nm - 800 nm bzw. 770 nm - 800 nm entsprechen. Anhand Fig. 7 wird deutlich, daß die Unterscheidung zwischen Karies und Zahnschmelz verbessert und der Quotient der Meßsignale für Karies und Zahnschmelz größer wird, wenn nur die längerwellige Fluoreszenzstrahlung (vgl. Kurven f) und g)) ausgenutzt wird.

Aufgrund der zuvor beschriebenen Beobachtungen wurden die Untersuchungen für noch größere Wellenlängen im Nahinfrarotbereich (NIR-Bereich) fortgesetzt, obwohl hier eigentlich im biologischen Bereich keine Fluoreszenzstrahlung mehr erwartet wird.

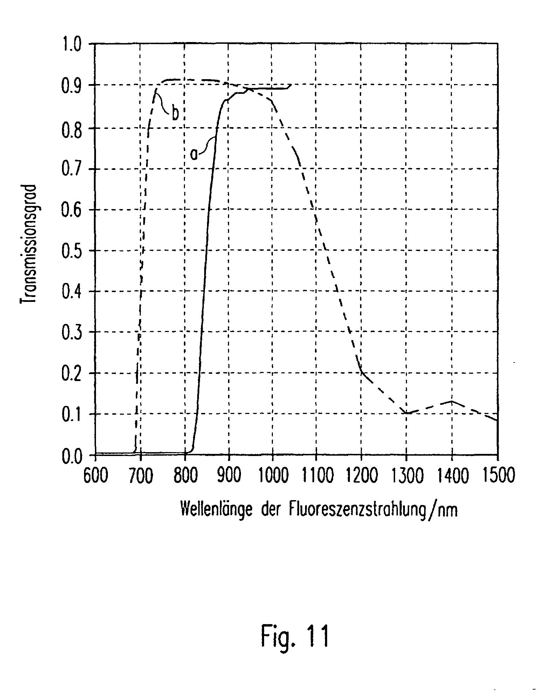

Zu diesem Zweck wurden einige Testmessungen mit einer bei einer Anregungswellenlänge von 780 nm emittierenden Laserdiode, die mit Hilfe eines Kurzpassfilters von langwelliger Untergrundstrahlung gereinigt wurde, und einem entsprechenden Kantenfilter, das für Wellenlängen der Fluoreszenzstrahlung größer als ca. 850 nm durchlässig ist, durchgeführt. Zu Vergleichszwecken wurde die aus der DE 19541686 A1 bekannte Kombination einer Anregungswellenlänge von ca. 655 nm und eines Spektralfilters, das für Fluoreszenzstrahlung mit einer Wellenlänge größer als 670 nm durchlässig ist, untersucht. Die Transmssionsgrade der bei den Messungen verwendeten Filter sind in Figur 11 dargestellt, wobei die Kurve a) den Transmissionsgrad des Kantenfilters mit dem Durchlaßbereich für Wellenlängen größer als ca. 850 nm und die Kurve b) den Transmissionsgrad des Spektralfilters mit dem Durchlaßbereich für Wellenlängen größer als ca. 670 nm darstellt.

Die im Laufe dieser Untersuchungen ermittelten Ergebnisse bestätigten, daß unter den zuvor erläuterten Bedingungen, d.h. bei einer Anregungswellenlänge von ca. 780 nm und einer Erfassung der Fluoreszenzstrahlung für Wellenlängen größer als ca. 850 nm (vgl. Kurve a) in Fig. 11), nahezu keine Fluoreszenzstrahlung von gesundem Zahnschmelz oder Dentin nachgewiesen werden konnte, während hingegen eine Fluoreszenzstrahlung von kariösen Bereichen erfaßt werden konnte. Die hierbei erfaßten Meßsignale sind zwar kleiner als bei der bisherigen Vorgehensweise, sie heben sich aber relativ zu dem sehr kleinen "Störsignal" vom gesunden Zahngewebe sehr gut ab, d. h. es besteht ein großes Verhältnis des Meßsignals für kariöse Bereiche zu dem Meßsignal für gesunde Zahnbereiche. Dadurch läßt sich die Empfindlichkeit des Detektors bei versteckter Karies deutlich steigern. Dies wurde an einer Zahnreihe mit Approximalkaries überprüft und bestätigt. Es wird vermutet, daß mit Hilfe der oben angegebenen Kombination einer Anregungswellenlänge von ca. 780 nm mit einer Auswertung der angeregten Fluoreszenzstrahlung für Wellenlängen größer als 850 nm ein Fluorophor nachgewiesen wird, welches sich nicht im gesunden Zahn befindet, wohl aber in Karies oder in Konkrementen und ähnlichen Bestandteilen enthalten ist, und möglicherweise einer organischen Einlagerung oder einem Bakterienstoffwechselprodukt entsprechen kann. Auf der anderen Seite lassen die spektralen Untersuchungen vermuten, das hauptsächlich Veränderungen an dem anorganischen Gewebegerüst detektiert werden.

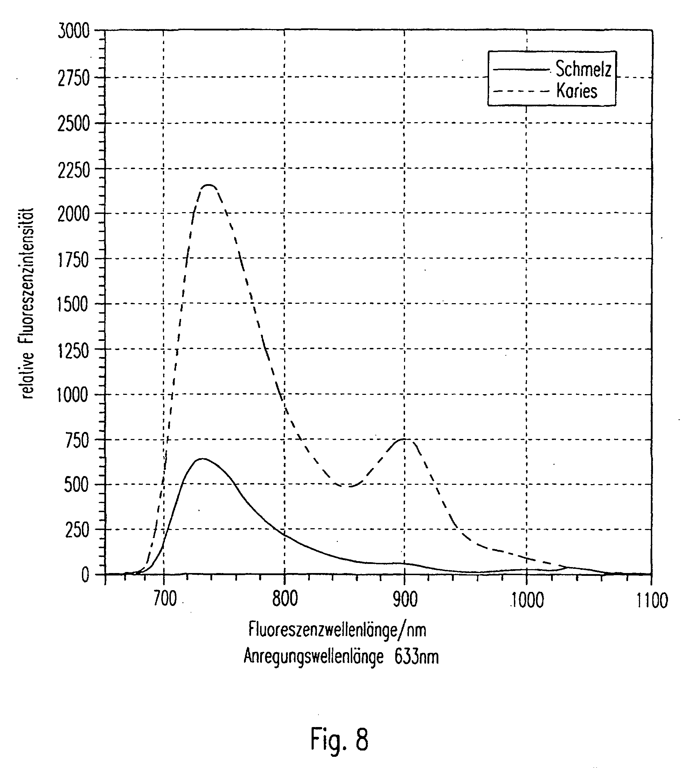

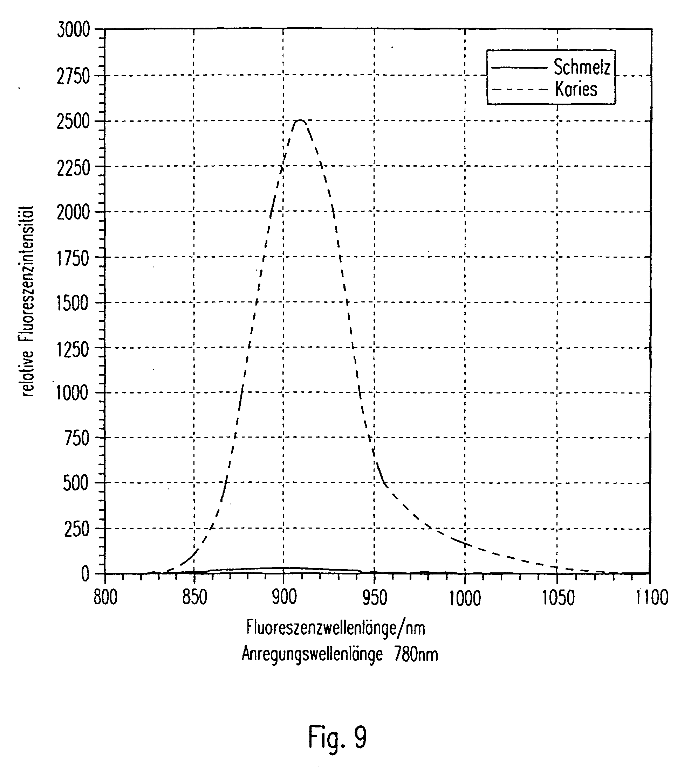

In Fig. 8 und 9 sind Meßergebnisse für entsprechende Untersuchungen für eine Anregungswellenlänge von 633 nm bzw. 780 nm dargestellt, wobei jeweils die erfaßte Fluoreszenzintensität für kariöse Zahnbereiche und Zahnschmelz abhängig von der Fluoreszenzwellenlänge aufgetragen ist. Aus Fig. 8 ist bei dem für Karies erfaßten Verlauf der Fluoreszenzstrahlung eine spezifische Emissionsbande um 900 nm ersichtlich, die dafür sorgt, daß sich im Fluoreszenzwellenlängenbereich über 850 nm die Fluoreszenzintensitäten für Karies und Zahnschmelz deutlicher unterscheiden als im gesamten Intervall ab 700 nm. Bei der in Fig. 9 gezeigten Anegungswellenlänge von 780 nm wird die gesamte Fluoreszenzemission bei kariösen Zahnbereichen lediglich durch diese eine Spitze (Peak) gebildet, während Zahnschmelz nahezu nicht mehr fluoreziert, so daß das Verhältnis der Fluorezenzintensitäten für Karies und Zahnschmelz für diese Anregungswellenlänge weiter vergrößert werden kann, um somit die Detektionsgenauigkeit zu verbessern.

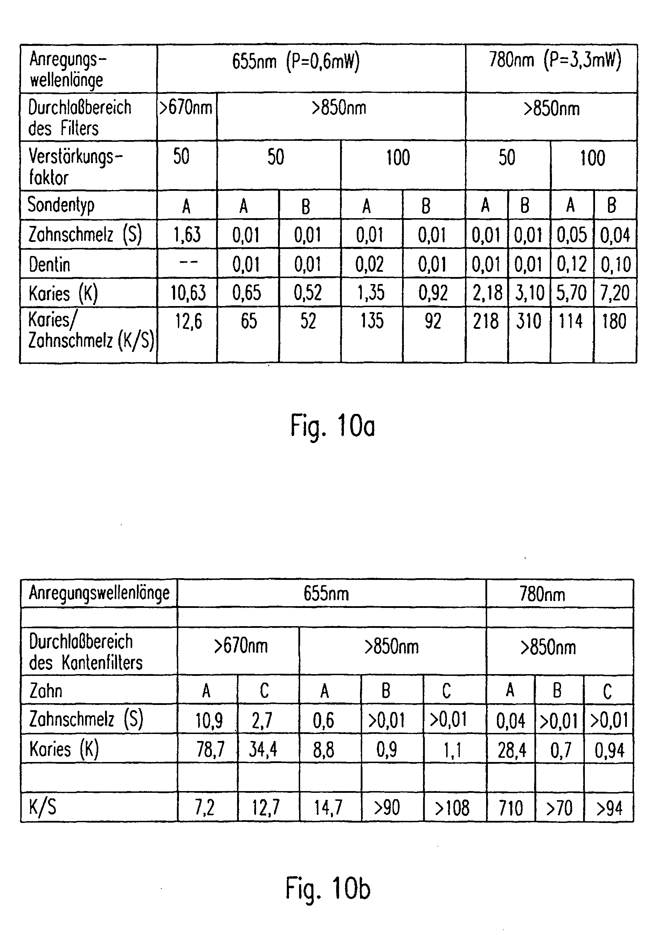

Weitere Aufschlüsse gaben Untersuchungen, deren Ergebnisse in Figur 10a und 10b dargestellt sind.

Dabei sind in Fig. 10a für unterschiedlichen Anregungswellenlängen 655 nm (Bestrahlungsleistung P=0,6 mW) und 780 nm (Bestrahlungsleistung P=3,3 mW), für unterschiedliche Grenzwellenlängen des jeweils verwendeten Spektralfilters und für unterschiedliche Verstärkungsfaktoren die dabei detektierte Intensität der Fluoreszenzstrahlung für gesunden Zahnschmelz, Dentin und kariöse Zahnbereiche dargestellt. Insbesondere sind in Fig. 10a die Meßergebnisse für zwei unterschiedliche Sondenarten (Typ A bzw. Typ B) aufgetragen. Ergänzend ist in der untersten Zeile von Fig. 10a für jede Kombination der jeweiligen Anregungswellenlänge mit dem entsprechenden Spektralfilter (Kantenfilter), dem entsprechenden Verstärkungsfaktor und der entsprechenden Sonde das Verhältnis der Fluoreszenzstrahlung des kariösen Bereichs zu der Fluoreszenzstrahlung des Zahnschmelzbereichs dargestellt, wobei ein hoher Verhältniswert einer hohen Detektionsempfindlichkeit entspricht, da sich in diesem Fall das Meßsignal für kariöse oder befallene Zahnbereiche deutlich von dem Meßsignal für gesunde Zahnbereiche unterscheidet.

Werden die in Fig. 10a dargestellten Resultate für die Anregungswellenlängen 655 nm und 780 nm bei jeweils gleichem Detektionsbereich für Fluoreszenzstrahlungswellenlängen größer als 850 nm miteinander verglichen, so entsprechen sich die dabei auftretenden Meßsignale näherungsweise, falls die Meßergebnisse auf dieselbe Bestrahlungsleistung umgerechnet werden. Dies ist somit ein Anzeichen dafür, daß das zuvor beschriebene Fluorophor bei beiden Wellenlängen in etwa gleich stark angeregt wird. Vergleicht man hingegen die in Figur 10a dargestellten Ergebnisse bei derselben Anregungswellenlänge von 655 nm, jedoch für unterschiedliche Spektralfilter, d. h. für Fluoreszenzstrahlungswellenlängen größer als 670 nm bzw. 850 nm, so wird deutlich, daß nahezu der gesamte Anteil des mit der aus der DE 19541686 A1 bekannten Kombination (Anregungswellenlänge 655 nm und Auswertung der Fluoreszenzstrahlung für Wellenlängen zwischen 670 nm und 800 nm) erhaltenen Meßsignals aus dem Wellenlängenbereich zwischen 700 nm und 850 nm stammt, d. h. dieser Anteil stammt nicht von dem zuvor beschriebenen, in besonderem Maße kariesspezifischen Fluorophor.

Fig. 10b zeigt entsprechende Meßergebnisse für unterschiedliche Zähne A, B und C, wobei die angegebenen Meßsignale auf die Leistung der Anregungsstrahlung normiert sind und jeweils der Quotient K/S aus der erfaßten Fluorezenzstrahlung für Karies (K) und Zahnschmelz (S) dargestellt ist. Man erkennt wiederum, daß sich bei allen Zähnen A, B, und C die erfaßten Fluorezenzstrahlungen für Karies und Zahnschmelz deutlicher voneinander unterscheiden, wenn der Nachweis bzw. die Detektion auf langwellige Fluoreszenzstrahlung, insbesondere auf Fluoreszenzwellenlängen größer als ca. 850 nm statt auf Fluoresezenzwellenlängen größer als ca. 670 nm, beschränkt wird. Bei dem Zahn A zeigt sich darüber hinaus ein deutlicher Vorteil, d.h. ein deutlich größerer Quotient K/S, für die größere Anregungswellenlänge 780 nm im Vergleich zu der Anregungswellenlänge 650 nm.

Nach den in Figur 5-10 dargestellten Meßergebnissen läßt sich eine besonders empfindliche Detektion von versteckter Karies, beispielsweise in Fissuren oder approximalen Zahnbereichen, gemäß der vorliegenden Erfindung erreichen, falls die Fluoreszenzstrahlung mit Wellenlängen oberhalb von ca. 800 nm nachgewiesen wird, d. h. der Nachweisbereich der Fluoreszenzstrahlung auf Wellenlängen größer als ca. 800 nm beschränkt wird, da in diesem Wellenlängenbereich der Fluoreszenzstrahlung kariesspezifische Fluorophore und vermutlich auch andere Ablagerungen fluoreszieren, nicht aber gesunder Zahnschmelz oder Dentin. Besonders vorteilhaft ist die Beschränkung des Nachweisbereiches auf Fluoreszenzwellenlängen größer als ca. 850 nm. Zur Anregung dieser Fluoreszenzstrahlung kommen im Prinzip alle Wellenlängen unterhalb von 800 nm in Betracht. Hinsichtlich der optischen Eindringtiefe ist jedoch wegen der mit zunehmender Wellenlänge abnehmenden Streuung einer Anregung mit möglichst langwelliger Strahlung vorteilhaft, so daß insbesondere Anregungswellenlängen größer als 680 nm, wie z. B. die zuvor angegebene Anregungswellenlänge 780 nm, in Frage kommt.

Da ober- und unterhalb einer Anregungswellenlänge offenbar verschiedene Fluorophore oder verschiedene Gruppen von Fluorophoren erfaßt werden, ist für eine verbesserte Differenzierung und Detektion (z. B. von Dekalzifizierungen und Läsionen mit organischen Einlagerungen oder bakteriellem Befall) auch die Kombination verschiedener Nachweisbereiche, d. h. verschiedener ausgewerteter Bereiche der Fluoreszenzstrahlungswellenlänge und/oder verschiedene Anregungswellenlängen vorteilhaft.

## Patentansprüche

1. Verfahren zum Messen der Fluoreszenzaktivität von Zähnen, umfassend die Schritte
a) Bestrahlen eines zu untersuchenden Zahns (4) mit einer Anregungsstrahlung (9), die an dem Zahn (4) eine Fluoreszenzstrahlung (10) hervorruft, und
b) Erfassen und Auswerten der am Zahn (4) durch die Anregungsstrahlung (9) hervorgerufenen Fluoreszenzstrahlung (10),
wobei im Schritt a) die Wellenlänge der Anregungstrahlung (9) zwischen 680 nm und 800 nm liegt, und
im Schritt b) die Fluoreszenzstrahlung (10) für Wellenlängen grösser als 850 nm ausgewertet wird.

2. Verfahren nach einem der vorhergehenden Ansprüche,
**dadurch gekennzeichnet,**
**dass** die Wellenlänge der Anregungsstrahlung (9) ca. 780 nm beträgt.

3. Verfahren nach Anspruch 1 oder 2,
**dadurch gekennzeichnet,**
**dass** der zu untersuchende Zahn (4) im Schritt a) mit Anregungsstrahlungen (9) unterschiedlicher Wellenlänge bestrahlt wird, und
**dass** im Schritt b) Fluoreszenzstrahlungen (10) unterschiedlicher Wellenlängen erfasst und ausgewertet werden.

4. Verfahren nach einem der vorhergehenden Ansprüche,
**dadurch gekennzeichnet,**
**dass** aus der Anregungsstrahlung eine langwellige Untergrundstrahlung ausgefiltert wird, bevor diese den zu untersuchenden Zahn (4) bestrahlt.

5. Vorrichtung zum Erkennen von Karies, Plaque, Konkrementen oder bakteriellem Befall an Zähnen,
mit einer Lichtquelle (1) zum Erzeugen einer Anregungsstrahlung (9), welche auf einen zu untersuchenden Zahn (4) zu richten ist und an dem Zahn (4) eine Fluoreszenzstrahlung (10) hervorruft,
mit einer Erfassungseinrichtung (8) zum Erfassen der Fluoreszenzstrahlung (10) des Zahnes (4), und
mit der Erfassungseinrichtung (8) vorgeschalteten spektralen Filtermitteln (7),
wobei die spektralen Filtermittel (7) derart ausgestaltet sind, dass sie Fluoreszenzstrahlung (10) mit einer Wellenlänge grösser als 850 nm durchlassen, und
die Lichtquelle (1) die Anregungsstrahlung (9) mit einer Wellenlänge zwischen 680 nm und 800 nm erzeugt.

6. Vorrichtung nach Anspruch 5,
**dadurch gekennzeichnet,**
**dass** die Lichtquelle (1) die Anregungsstrahlung (9) mit einer Wellenlänge von ca. 780 nm erzeugt.

7. Vorrichtung nach einem der Ansprüche 5 und 6,
**dadurch gekennzeichnet,**
**dass** die spektralen Filtermittel (7) ein Farbglas-Kantenfilter umfassen.

8. Vorrichtung nach einem der Ansprüche 5 bis 7,
**dadurch gekennzeichnet,**
**dass** die spektralen Filtermittel (7) ein schwach eigenfluoreszierendes Spektralfilter umfassen.

9. Vorrichtung nach einem der Ansprüche 5 bis 8,
**dadurch gekennzeichnet,**
**dass** die Erfassungseinrichtung (8) eine Auswertungseinrichtung umfasst, welche aufgrund der durch die Erfassungseinrichtung (8) detektierten Fluoreszenzstrahlung (10) des Zahnes (4) auf das Vorhandensein bzw. Nichtvorhandensein eines gesunden oder kranken Zahnbereiches schliesst.

10. Vorrichtung nach einem der Ansprüche 5 bis 9,
**dadurch gekennzeichnet,**
**dass** eine Lichtleitfaser (13) zur Übertragung der Anregungsstrahlung (9) vorgesehen ist, deren zahnseitiges Ende abgeschrägt und verspiegelt ist, so dass die Anregungstrahlung (9) seitlich aus der Lichtleitfaser (13) austritt.

11. Vorrichtung nach einem der Ansprüche 5 bis 10,
**dadurch gekennzeichnet,**
**dass** ein Lichtkeil (3) zur Bestrahlung des Zahnes (4) mit der Anregungsstrahlung (9) vorgesehen ist.

12. Vorrichtung nach einem der Ansprüche 5 bis 11,
**dadurch gekennzeichnet,**
**dass** ein gemeinsamer Lichtleiter (3) für die Übertragungder Anregungsstrahlung (9) und der Fluoreszenzstrahlung (10) vorgesehen ist.

13. Vorrichtung nach Anspruch 12,
**dadurch gekennzeichnet,**
**dass** der Lichtleiter (3) mindestens eine Lichtleitfaser (13) zur Übertragung der Anregungsstrahlung (9) und mehrere konzentrisch angeordnete Lichtleitfasern (14) zur Übertragung der Fluoreszenzstrahlung (10) aufweist.

14. Vorrichtung nach Anspruch 13,
**dadurch gekennzeichnet,**
**dass** die spektralen Filtermittel (7) einen Filterglasring (15) umfassen, der zwischen den konzentrisch angeordneten Lichtleitfasern (14) zur Übertragung der Fluoreszenzstrahlung (10) und der Erfassungseinrichtung (8) angeordnet ist.

15. Vorrichtung nach einem der Ansprüche 12 bis 14,
**dadurch gekennzeichnet,**
**dass** zwischen der Lichtquelle (1) und dem lichtquellenseitigen Ende des gemeinsamen Lichtleiters ein Strahlteiler (11) zum Auskoppeln der Fluoreszenzstrahlung (10) vorgesehen ist, so dass die Erfassungseinrichtung (8) die aus dem gemeinsamen Lichtleiter (3) ausgekoppelte Fluoreszenzstrahlung (10) erfassen kann.

16. Vorrichtung nach Anspruch 12 oder 15,
**dadurch gekennzeichnet,**
**dass** in dem gemeinsamen Lichtleiter (3) die mindestens eine Lichtleitfaser (13) zur Übertragung der Anregungsstrahlung (9) und mindestens eine Lichtleitfaser (14) zur Übertragung der Fluoreszenzstrahlung (10) am zahnseitigen Ende des Lichtleiter (3) linear nebeneinander angeordnet sind.

17. Vorrichtung nach einem der Ansprüche 5 bis 16,
**dadurch gekennzeichnet,**
**dass** Verstärkermittel zur Verstärkung der Fluoreszenzstrahlung (10) des Zahnes (4) vorgesehen sind, um der Erfassungseinrichtung (8) die verstärkte Fluoreszenzstrahlung (10) zuzuführen.

18. Vorrichtung nach einem der Ansprüche 5 bis 17,
**dadurch gekennzeichnet,**
**dass** zwischen der Lichtquelle (1) und dem zu untersuchenden Zahn (4) weitere spektrale Filtermittel (12) angeordnet sind, welches derart ausgestaltet sind, dass sie aus der Anregungsstrahlung eine langwellige Untergrundstrahlung ausfiltern.

## Claims

1. Method for measuring the fluorescence activity of teeth, including the steps
a) irradiation of a tooth (4) to be investigated with an excitation radiation (9) which brings about a fluorescence radiation (10) at the tooth (4), and
b) detection and evaluation of the fluorescence radiation (10) brought about at the tooth (4) by the excitation radiation (9),
wherein
in step a) the wavelength of the excitation radiation (9) lies between 680 nm and 800 nm, and
in step b) the fluorescence radiation (10) is evaluated for wavelengths greater than 850 nm.

2. Method according to any preceding claim,
**characterised in that**,
the wavelength of the excitation radiation (9) is ca. 780 nm.

3. Method according to claim 1 or 2,
**characterised in that**,
in step a) the tooth (4) to be investigated is irradiated with excitation radiations (9) of different wavelengths, and
**in that** in step b) fluorescence radiations (10) of different wavelengths are detected and evaluated,

4. Method according to any preceding claim,
**characterised in that**,
a long wavelength background radiation is filtered out of the excitation radiation before this irradiates the tooth to be investigated (4).

5. Device for the recognition of caries, plaque, concretions or bacterial infection on teeth,
having a light source (1) for the generation of an excitation radiation (9), which is to be directed at a tooth (4) to be investigated and which brings about a fluorescence radiation (10) at the tooth (4),
having a detection device (8) for the detection of the fluorescence radiation (10) of the tooth (4), and
having spectral filter means (7) arranged before the detection device (8),
wherein
the spectral filter means (7) are so configured that they let through fluorescence radiation (10) having a wavelength greater than 850nm, and
the light source generates the excitation radiation (9) with a wavelength between 680 nm and 800 nm.

6. Device according to claim 5,
**characterised in that**,
the light source (1) generates the excitation radiation (9) with a wavelength of ca. 780 nm.

7. Device according to any of claims 5 and 6,
**characterised in that**,
the spectral filter means (7) includes a coloured glass cut-off filter.

8. Device according to any of claims 5 to 7,
**characterised in that**,
the spectral filter means (7) includes a weakly self-fluorescing spectral filter.

9. Device according to any of claims 5 to 8,
**characterised in that**,
the detection device (8) includes an evaluation device which on the basis of the fluorescence radiation (10) of the tooth (4) detected by means of the detection device (8) determines upon the presence or non-presence of a healthy or diseased tooth region.

10. Device according to any of claims 5 to 9,
**characterised in that**,
there is provided a light conductor fibre (13) for the transmission of the excitation radiation (9), the end of which towards the tooth is chamfered and made reflective, so that the excitation radiation (9) emerges from the light conductor fibre (13) laterally.

11. Device according to any of claims 5 to 10,
**characterised in that**,
there is provided a light wedge (3) for the irradiation of the tooth (4) with the excitation radiation (9).

12. Device according to any of claims 5 to 11,
**characterised in that**,
there is provided a common light conductor (3) for the transmission of the excitation radiation (9) and of the fluorescence radiation (10).

13. Device according to claim 12,
**characterised in that**,
the light conductor (3) has at least one light conductor fibre (13) for the transmission of the excitation radiation (9) and a plurality of concentrically arranged light conductor fibres (14) for the transmission of the fluorescence radiation (10).

14. Device according to claim 13,
**characterised in that**,
the spectral filter means (7) includes a filter glass ring (15) which is arranged between the concentrically arranged light conductor fibres (14) for the transmission of the fluorescence radiation (10) and the detection device (8).

15. Device according to any of claims 12 to 14,
**characterised in that**,
between the light source (1) and the end of the common light conductor towards the light source there is provided a beam divider (11) for coupling out the fluorescence radiation (10), so that the detection device (8) can detect the fluorescence radiation (10) coupled out of the common light conductor (3).

16. Device according to any of claims 12 or 14,
**characterised in that**,
in the common light conductor (3) the at least one light conductor fibre (13) for the transmission of the excitation radiation (9) and at least one light conductor fibre (14) for the transmission of the fluorescence radiation (10) are arranged linearly next to one another at the end of the light conductor (3) towards the tooth.

17. Device according to any of claims 5 to 16,
**characterised in that**,
amplifier means for the amplification of the fluorescence radiation (10) of the tooth (4) are provided, in order to supply the amplified fluorescence radiation (10) to the detection device (8).

18. Device according to any of claims 5 to 17,
**characterised in that**,
there are arranged between the light source (1) and the tooth (4) to be investigated further spectral filter means (12) which are so configured that they filter out of the excitation radiation a long wavelength background radiation.

## Revendications

1. Procédé de mesure de l'activité de fluorescence des dents, comprenant les étapes de :
a) irradiation d'une dent (4) à examiner, avec un rayonnement d'activation (9), qui engendre un rayonnement de fluorescence (10) sur la dent (4), et
b) détection et évaluation du rayonnement de fluorescence (10) engendré sur la dent (4) par le rayonnement d'activation (9),
où à l'étape a), la longueur d'onde du rayonnement d'activation (9) se situe entre 680 nm et 800 nm, et
à l'étape b), le rayonnement de fluorescence (10) est évalué à des longueurs d'onde supérieures à 850 nm.

2. Procédé selon la revendication précédente, **caractérisé en ce que** la longueur d'onde du rayonnement d'activation (9) se situe à environ 780 nm.

3. Procédé selon la revendication 1 ou 2, **caractérisé en ce que** la dent (4) à examiner est irradiée à l'étape a), avec des rayonnements d'activation (9) de différentes longueurs d'onde, et que à l'étape b), on détecte et évalue des rayonnements de fluorescence (10) de différentes longueurs d'onde.

4. Procédé selon l'une quelconque des revendications précédentes, **caractérisé en ce qu'**un rayonnement de base à longue longueur d'onde est filtré du rayonnement d'activation, avant que celui-ci irradie la dent (4) à examiner.

5. Dispositif destiné à la détection des caries, de la plaque, de concrétions ou d'affections bactériennes sur des dents,
avec une source lumineuse (1) pour produire un rayonnement d'activation (9), qui est dirigé sur une dent (4) à examiner et engendre sur la dent (4) un rayonnement de fluorescence (10),
avec un dispositif de détection (8) pour détecter la rayonnement de fluorescence (10) de la dent (4), et
avec des filtres spectraux (7) disposés avant le dispositif de détection (8),
où les filtres spectraux (7) sont équipé de sorte qu'ils laissent passer le rayonnement de fluorescence (10) ayant une longueur d'onde supérieure à 850 nm, et que la source lumineuse (1) produit le rayonnement d'activation (9) avec une longueur d'onde allant de 680 nm à 800 nm.

6. Dispositif selon la revendication 5, **caractérisé en ce que** la source lumineuse (1) produit un rayonnement d'activation (9) avec une longueur d'onde d'environ 780 nm.

7. Dispositif selon l'une quelconque des revendications 5 et 6, **caractérisé en ce que** les filtres spectraux (7) comprennent un bord de filtre en verre coloré.

8. Dispositif selon l'une quelconque des revendications 5 à 7, **caractérisé en ce que** les filtres spectraux (7) comprennent un filtre spectral à fluorescence propre faible.

9. Dispositif selon l'une quelconque des revendications 5 à 8, **caractérisé en ce que** le dispositif de détection (8) comprend un dispositif d'évaluation, qui conclut à la présence ou la non-présence d'une zone saine ou malade de la dent sur base du rayonnement de fluorescence (10) de la dent (4), détecté par le dispositif de détection (8).

10. Dispositif selon l'une quelconque des revendications 5 à 9, **caractérisé en ce qu'**une fibre optique (13) est prévue pour la transmission du rayonnement d'activation (9), dont l'extrémité du côté de la dent est biseautée et métallisée de sorte que le rayonnement d'activation (9) sort latéralement de la fibre optique (13).

11. Dispositif selon l'une quelconque des revendications 5 à 10, **caractérisé en ce qu'**un coin lumineux (3) est prévu pour l'irradiation de la dent (4) avec le rayonnement d'activation (9).

12. Dispositif selon l'une quelconque des revendications 5 à 11, **caractérisé en ce qu'**un conducteur de lumière commun (3) est prévu pour la transmission du rayonnement d'activation (9) et du rayonnement de fluorescence (10).

13. Dispositif selon la revendication 12, **caractérisé en ce que** la conducteur de lumière (3) présente au moins une fibre optique (13) pour la transmission du rayonnement d'activation (9) et plusieurs fibres optiques (14) disposées de manière concentrique, pour la transmission du rayonnement de fluorescence (10).

14. Dispositif selon la revendication 13, **caractérisé en ce que** les filtres spectraux (7) comprennent un anneau en verre de filtre (15), qui est disposé entre les fibres optiques (14) disposées de manière concentrique, pour la transmission du rayonnement de fluorescence (10) et le dispositif de détection (8).

15. Dispositif selon l'une quelconque des revendications 12 à 14, **caractérisé en ce que** entre la source lumineuse (1) et l'extrémité du côté de la source lumineuse du conducteur lumineux commun, est prévue une lame séparatrice (11) pour le couplage du rayonnement de fluorescence (10), de sorte que le dispositif de détection (8) peut détecter le rayonnement de fluorescence (10) couplé au conducteur de lumière (3) commun.

16. Dispositif selon l'une quelconque des revendications 12 ou 15, **caractérisé en ce que** dans le conducteur de lumière (3) commun, au moins une fibre optique (13) pour la transmission du rayonnement d'activation (9) et au moins une fibre optique (14) pour la transmission du rayonnement de fluorescence (10) sont disposées sur l'extrémité côté dent du conducteur de lumière (3), de manière linéaire l'un à côté de l'autre.

17. Dispositif selon l'une quelconque des revendications 5 à 16, **caractérisé en ce que** des agents de renforcement sont prévus pour renforcer le rayonnement de fluorescence (10) de la dent (4), pour conduire au dispositif de détection (8), le rayonnement de fluorescence (10) renforcé.

18. Dispositif selon l'une quelconque des revendications 5 à 17, **caractérisé en ce que** entre la source lumineuse (1) et la dent (4) à examiner sont disposés d'autres filtres spectraux (12), qui sont équipés de sorte qu'ils filtrent un rayonnement de base de longue longueur d'onde du rayonnement d'activation.
